# EUROPEAN PATENT APPLICATION

(11) **EP 2 238 948 A1**
(43) Date of publication of application: **13.10.2010**
(21) Application number: 10153200.0
(22) Date of filing: 10.02.2010
(51) Int. Cl.: A61F 2/30, A61F 2/46

(54) **Bone graft shaping system and method using the same**

(30) Priority: 01.04.2009 KR 20090027998
(71) Applicant: National Cancer Center, Ilsandong-gu Goyang-si, Gyeonggi-do (KR)
(72) Inventor: Kim, Kwang-Gi, Gyeonggi-do (KR); Kang, Hyun-Guy, Gyeonggi-do (KR)
(74) Representative: Schoppe, Fritz

(57) **Abstract**

Provided are a bone graft shaping system and method that are capable of precisely shaping a bone graft to compactly couple the bone graft to a bone area from which an affected part is removed during bone graft surgery. The bone graft shaping system includes a profile measuring part configured to measure a profile of a bone graft and a profile of a bone from which an affected part is removed to generate three-dimensional profile information, a shaping position generator configured to compare and analyze a shaping position of the bone graft corresponding to the bone from which the affected part is removed according to the generated three-dimensional profile information to generate shaping position information, a shaping profile generator configured to generate shaping profile information to shape the bone graft according to the three-dimensional profile information corresponding to the bone from which the affected part is removed, a shaping processor configured to shape the bone graft, and a controller configured to control the shaping processor on the basis of the three-dimensional profile information, the shaping position information, and the shaping profile information.

## Description

### CROSS-REFERENCE TO RELATED APPLICATION

This application claims the benefit of Korean Patent Application No. 2009-27998, filed April 1, 2009, the disclosure of which is hereby incorporated herein by reference in its entirety.

### BACKGROUND

### 1. Field of the Invention

The present invention relates to a bone graft shaping system and method, and more particularly, to a bone graft shaping system and method that are capable of precisely shaping a bone graft to compactly couple the bone graft to a bone area from which an affected part is removed, during bone graft surgery.

### 2. Discussion of Related Art

In general, a bone graft is used when a bone is seriously injured. Here, the serious injury refers to a case in which a portion of the bone is lost or must be removed due to a tumor, serious degenerative or rheumatic arthritis, deformity, serious osteoporosis, an injury, an accident, etc.

Bone graft surgery is a surgical operation or technique in which the removed bone is filled with the bone graft to form its original shape.

The bone graft may be extracted from the body of the patient himself/herself (autograft), may be extracted from the bone of a donor (allograft), or may be formed of an artificial bone.

In addition, in order to successfully recover functions of the patient's bone using the bone graft, the bone graft must be securely and rigidly adhered to the patient's bone.

The autograft or the allograft may be classified into a morselized bone graft or a strut bone graft. In the morselized bone graft, collected bones are morselized to be used, while in the strut bone graft, the bone graft is used without being morselized to maintain the shape and structure of the area having the removed bone.

The strut bone graft is used in an area in which the bone is injured to a wide extent or a large weight load is applied, and mainly used in the case that musculoskeletal structures such as joints, ligaments and tendons around the joints, etc, must be simultaneously recovered.

When a large extent of bone injury occurs due to a bone tumor, various diseases, accidents, etc., tumor prosthesis, arthroplasty, etc., have mainly been used so far. However, since the lifespan of metal inserts is limited and the metal inserts are permanently present in the body, many side effects occur, requiring several re-operations.

While a strut bone autograft is most ideal for the bone graft, since a size in collection is limited and a normal area may be injured, it is difficult to use in practice. In this state, since the allograft collected from the donor's bone can minimize use of inner metal fixtures and injury caused by collection of bone, the allograft has been widely used. In recent times, developments in basic science have led to research into development of artificial bones which are expected to lead the autograft and allograft to solve the above problems.

Use of the allograft enables surgery capable of preserving important bone structures such as joints, growth plates of children, etc., minimizes use of artificial joints and inner metal structures, and removes necessity of re-operations through bone incorporation when there is no specific complication caused by the allograft such as inflammation, infection, injury, etc.

However, even though the allograft has various advantages, many limitations make the allograft difficult to use in actual surgeries. That is, since the bone graft cannot be formed and prepared to fit the injured area of the bone, and a surgeon must manually operate to fit the bone graft to the affected area, an operation time may be increased, serious surgical error may make the prepared bone graft unusable, and the manually operated bone graft may not precisely match with the removed bone.

Conventionally, since the bone grows very slowly, when a cracked gap of the bone is smaller than 0.3 mm, its growth is retarded, and when the gap is larger than 2 mm, it is difficult for the bone to grow to adhere the gap.

Therefore, while the bone graft must be formed not to depart from the range of 0.3 to 2 mm, it is substantially impossible to precisely shape the whole surface of the bone graft within the range of 0.3 to 2 mm manually during the operation.

In spite of the above problems, in the case of bone tumors, serious diseases or deformities, there are many necessary cases of recovering functions of the bone using the bone graft. However, the above problems still exist in spite of continuous attempts to solve them. Even if use of the artificial bones and the allograft are generalized, difficulties of manual operation of the bone graft may cause limitations in use.

### SUMMARY OF THE INVENTION

The present invention is directed to a bone graft shaping system and method that are capable of precisely shaping a bone graft to provide a predetermined gap for bone growth to a portion of a bone from which an affected part is removed, allowing normal growth of the bone.

In addition, the present invention is directed to a bone graft shaping system and method that are capable of precisely shaping the bone graft to anatomically stably fix the bone graft to the bone from which the affected part is removed, and minimizing insertion of artificial bones and inner metal fixtures.

Further, the present invention is directed to a bone graft shaping system and method that are capable of more precisely and rapidly shaping the bone graft to reduce an operation time.

Additional aspects of the invention will be set forth in the description which follows, and in part will be apparent from the description, or may be learned by practice of the invention.

In an example embodiment, a bone graft shaping system includes: a profile measuring part configured to measure a profile of a bone graft and a profile of a bone from which an affected part is removed to generate three-dimensional profile information; a shaping position generator configured to compare and analyze a shaping position of the bone graft corresponding to the bone from which the affected part is removed according to the generated three-dimensional profile information to generate shaping position information; a shaping profile generator configured to generate shaping profile information to shape the bone graft according to the three-dimensional profile information corresponding to the bone from which the affected part is removed; a shaping processor configured to shape the bone graft; and a controller configured to control the shaping processor on the basis of the three-dimensional profile information, the shaping position information, and the shaping profile information.

The profile measuring part may be a three-dimensional scanner using a laser.

The shaping processor may include a fixing member configured to fix the bone graft; and at least one cutting mechanism installed at a multi-joint robot arm to cut and shape the bone graft.

The system may further include a storage configured to store the three-dimensional profile information, the shaping position information, and the shaping profile information.

The system may further include a display configured to display at least one of the three-dimensional profile information, the shaping position information, and the shaping profile information on a screen.

The system may further include a sterilizer configured to sterilize the bone graft and the bone from which the affected part is removed.

The system may further include an abnormality detector configured to measure the sterilized bone graft to detect whether the bone graft is normal or not.

In another example embodiment, a bone graft shaping method using a bone graft shaping system includes: measuring profiles of a bone from which an affected part is removed and a sterilized bone graft, and generating three-dimensional profile information; storing the generated three-dimensional profile information in a storage; comparing and analyzing a shaping position of the bone graft corresponding to the bone from which the affected part is removed according to the stored three-dimensional profile information, and generating shaping position information; generating shaping profile information to shape the bone graft according to the three-dimensional profile information corresponding to the bone from which the affected part is removed; and shaping the bone graft on the basis of the shaping position information and the shaping profile information.

The method may further include inspecting the interior and exterior of the sterilized bone graft to determine whether the bone graft is normal or not, before generating the three-dimensional profile information.

### BRIEF DESCRIPTION OF THE DRAWINGS

The above and other objects, features and advantages of the present invention will become more apparent to those of ordinary skill in the art by describing in detail example embodiments thereof with reference to the attached drawings, in which:
FIG. 1 is a schematic view of a bone graft shaping system in accordance with an example embodiment of the present invention;
FIG. 2 is a block diagram schematically showing the constitution of the bone graft shaping system in accordance with an example embodiment of the present invention; and
FIG. 3 is a flowchart for explaining a bone graft shaping method using the bone graft shaping system in accordance with an example embodiment of the present invention.

### DETAILED DESCRIPTION OF EXAMPLE EMBODIMENTS

Hereinafter, example embodiments of the present invention will be described in detail. However, the present invention is not limited to the embodiments disclosed below, but can be implemented in various forms. The following embodiments are described in order to enable those of ordinary skill in the art to embody and practice the present invention. Like reference numerals designate like elements throughout the detailed description.

Hereinafter, a bone graft shaping system and method in accordance with an example embodiment of the present invention will be described with reference to the accompanying drawings. In the detailed description, if it is determined that description of conventional functions or constitutions may make the sprit of the invention unclear, detailed descriptions thereof will be omitted.

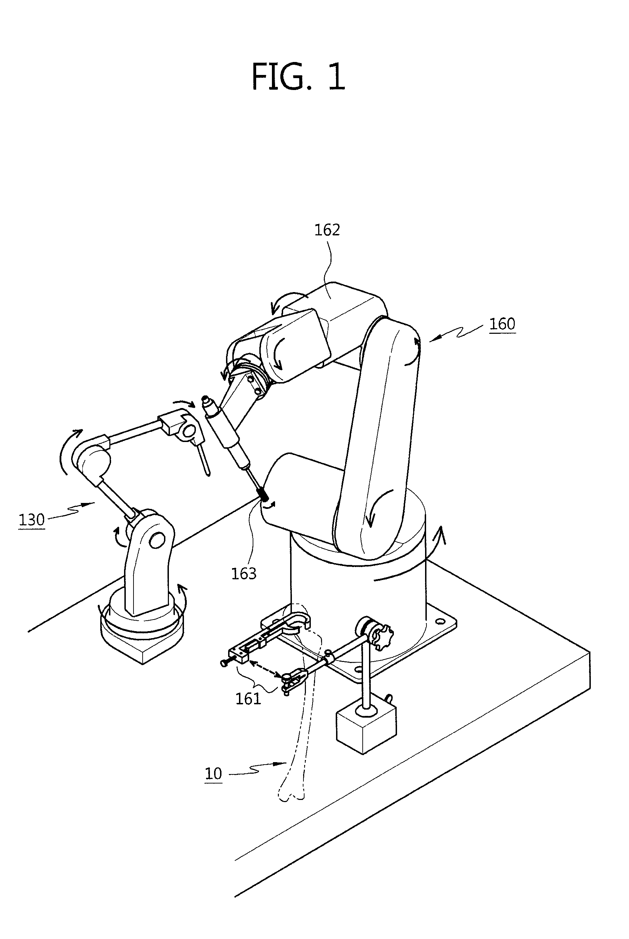

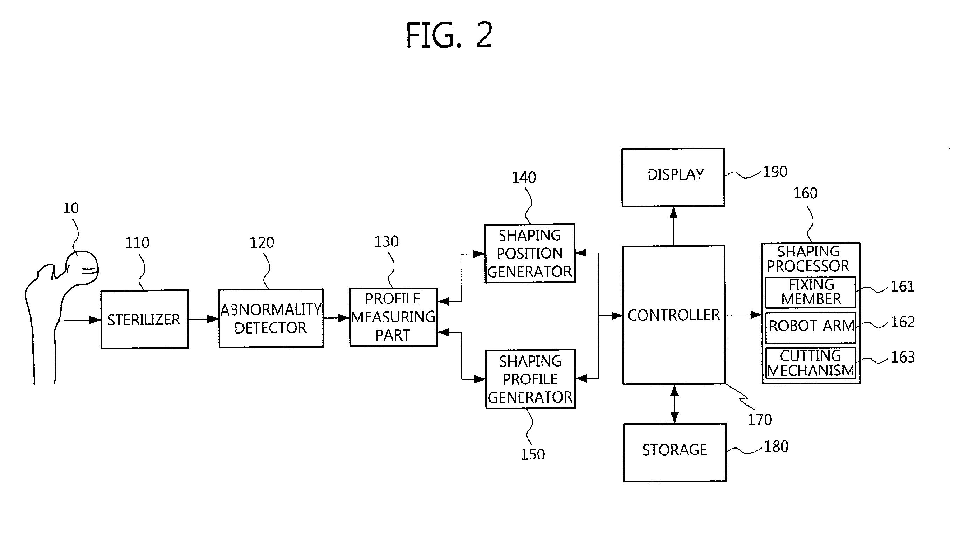

FIG. 1 is a schematic view of a bone graft shaping system in accordance with an example embodiment of the present invention, and FIG. 2 is a block diagram schematically showing the constitution of the bone graft shaping system in accordance with an example embodiment of the present invention.

Referring to FIGS. 1 and 2, the bone graft shaping system in accordance with an example embodiment of the present invention includes a sterilizer 110, an abnormality detector 120, a profile measuring part 130, a shaping position generator 140, a shaping profile generator 150, a shaping processor 150, a controller 170, a storage 180, and a display 190.

The sterilizer 110 is configured to sterilize various bacteria, viruses, and other infection sources of a bone graft 10, which is to be grafted to a patient's bone. That is, since the bone graft 10 is formed of a patient's own bone, or from that of another patient or a donor, it will be apparent to those skilled in the art that the bone graft must be sterilized, and thus detailed descriptions thereof will be omitted. Sterilization of the bone graft 10 may be performed by transilluminating γ-rays to sterilize the bacteria, viruses, and other infection sources.

In addition, the bone graft 10 may be, of course, sterilized by various methods including the sterilization using γ-rays.

The abnormality detector 120 is provided to precisely measure the interior and exterior of the sterilized bone graft 10 to determine whether the bone graft is normal or not, and configured to inspect a bone density, etc., to exclude use of an abnormal bone graft 10. Here, in order to inspect the bone graft 10, a conventional computed tomography apparatus may be used.

The computed tomography apparatus is conventionally referred to as a CT apparatus. Here, the CT apparatus functions to calculate and reconstruct an inner structure of an object on the basis of views projected in various directions. The CT apparatus may be an X-ray CT apparatus using X-ray transillumination, RI, a radiation CT apparatus using positron emission, a nuclear magnetic resonance therapy (NMRT) CT apparatus using nuclear magnetic resonance, a heavy particle beam CT apparatus, an ultrasonic CT apparatus, etc.

The profile measuring part 130 measures the bone graft 10 determined as a normal bone graft by the abnormality detector 120 and the bone from which an affected part is removed to provide a three-dimensional profile. At this time, three-dimensional profile information corresponding to the bone graft 10 and the bone from which the affected part is removed are generated.

The three-dimensional profile information may be selectively measured as the entire profile of the bone graft and a portion or the entire profile of the bone from which the affected part is removed.

In addition, the three-dimensional profile information is comparative analysis information for shaping the bone graft in a profile corresponding to the bone from which the affected part is removed.

Further, the profile measuring part 130 may be a well-known three-dimensional laser scanner, etc. In general, the three-dimensional laser scanner is excellent for a complicated three-dimensional free curve as a non-contact scan method using a laser displacement sensor.

The shaping position generator 140 receives the respective three-dimensional profile information to compare and analyze a shaping position of the bone graft 10 corresponding to the bone from which the affected part is removed on the basis of the respective three-dimensional profile information, thereby generating shaping position information.

Furthermore, the shaping position information may be configured to determine a shaping position using a self-programmed matching system on the basis of the respective three-dimensional profile information.

The shaping profile generator 150 generates shaping profile information, which can be coupled to the bone from which the affected part is removed on the basis of the position of the shaping position information and the three-dimensional profile information corresponding to the bone from which the affected part is removed provided from the profile measuring part 130.

Here, the shaping profile information is provided to shape the bone graft 10 to the bone from which the affected part is removed and correspond or conform to a peripheral profile of the bone.

The shaping processor 160 includes a fixing member 161, a multi-joint robot-arm 162, and at least one cutting mechanism 163 mounted on an end of the robot arm 162.

The shaping processor 160 shapes the bone graft 10 on the basis of the shaping position information and the shaping profile information provided from the controller 170, which will be described below.

Here, when the bone graft 10 is fixed to the fixing member 161 and the robot arm 162 moves on the basis of the shaping position information, the cutting mechanism 163 mounted on the end of the robot arm 162 moves to the shaping position of the bone graft 10.

Next, when a drive force is applied to the cutting mechanism 163, the cutting mechanism 163 is rotated at a high speed to precisely shape the bone graft 10 in a profile corresponding or confirming to the shaping profile information.

Here, the cutting mechanism 163 may include a milling cutter using a high speed rotating force.

In addition, the shaping processor 160 may use a conventional operation robot.

The controller 170 receives the three-dimensional profile information from the profile measuring part 130, the shaping position information from the shaping position generator 140, and the shaping profile information from the shaping profile generator 150 to control input/output thereof, and provides the shaping position information and the shaping profile information to the shaping processor 160 upon shaping of the bone graft 10 to control operations of the shaping processor 160 on the basis of the provided information.

Further, the controller 170 is electrically connected to the storage 180 and the display 190, and provides the three-dimensional profile information, the shaping position information and the shaping profile information to the storage 180 to store the three-dimensional profile information, the shaping position information and the shaping profile information therein.

Furthermore, the controller 170 also functions to output at least one of the three-dimensional profile information, the shaping position information and the shaping profile information stored in the storage 180 through the display 190.

Moreover, the display 190 is provided to output any one of the three-dimensional profile information, the shaping position information and the shaping profile information output from the controller 170 so that an operator can directly check and review the respective information to cope with various circumstances such as errors, etc.

Since the storage 180 and the display 190 are well-known techniques in the art, detailed descriptions thereof will be omitted.

As described above, when the bone graft 10 is shaped through the bone graft shaping system capable of precisely shaping the bone graft, the bone graft 10 may be coupled to the bone from which the affected part is removed through insertion, etc.

In addition, since the precise shaping can be performed on the basis of the three-dimensional profile information, the shaping position information and the shaping profile information, it is possible to shape the bone graft 10 having a predetermined gap of 0.3 to 2 mm, i.e., an allowable tolerance, without retarding the growth of the bone.

Describing an operation process of grafting the bone graft 10 to the bone using the bone graft shaping system, first, the bone graft 10 is prepared to be grafted to the bone before operation, after sterilizing the bone graft 10 of various bacteria and infection sources.

The abnormality detector 120 inspects the interior and exterior of the sterilized bone graft 10 using the CT apparatus to determine whether the bone graft 10 is normal or not through various inspection processes including bone density inspection, etc., thereby excluding use of the abnormal bone graft 10.

Meanwhile, in order to remove the affected part in the patient's bone, the skin tissue is incised and the affected part of the bone is removed.

In addition, the three-dimensional profile information corresponding to the bone graft 10 which is determined as normal by the abnormality detector 120, the bone graft 10 which is measured by the profile measuring part 130 with respect to the bone from which the affected part is removed, and the bone from which the affected part is removed, is generated.

The shaping position generator 140 analyzes a shaping position of the bone from which the affected part is removed to generate shaping position information of the bone graft 10 on the basis of the three-dimensional profile information.

The shaping profile generator 150 generates shaping profile information to couple the bone graft 10 to the bone from which the affected part is removed on the basis of the shaping position information and the three dimensional profile information of the bone from which the affected part is removed.

The shaping processor 160 performs a shaping operation of the bone graft 10 on the basis of the shaping profile information and the shaping position information.

The shaped bone graft 10 is sterilized by the sterilizer 110. At this time, the sterilization is performed by cleaning the bone graft 10 using a sterilized saline solution.

The sterilized bone graft 10 is coupled to the bone from which the affected part is removed, and the incised skin is sutured to complete the operation.

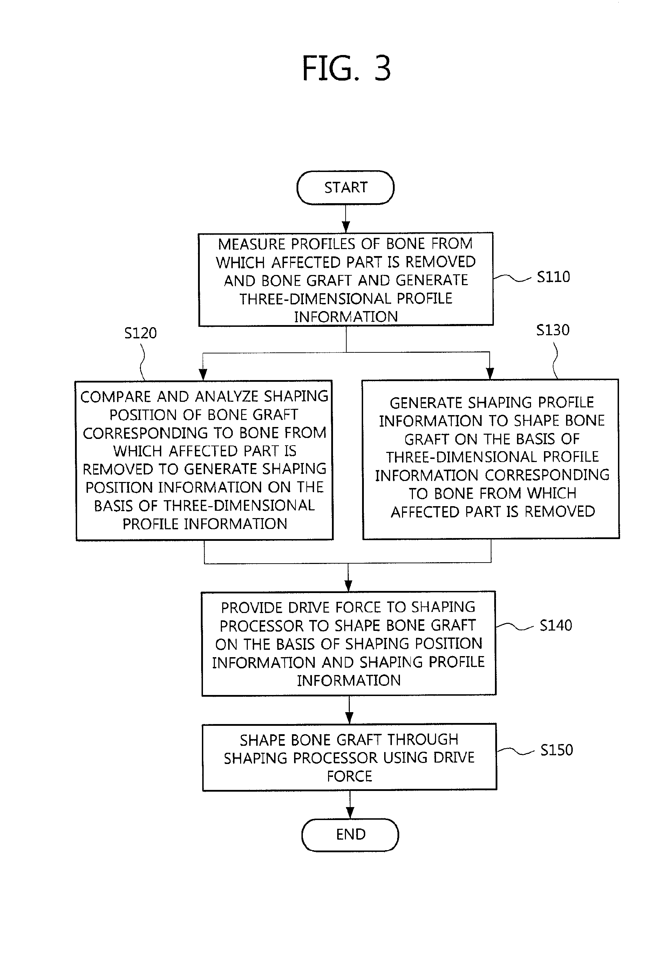

FIG. 3 is a flowchart for explaining a bone graft shaping method using the bone graft shaping system in accordance with an example embodiment of the present invention.

As shown in FIG. 3, the system in accordance with the present invention measures shapes of a sterilized bone graft and a bone from which an affected part is removed using a profile measuring part to generate the three-dimensional profile information (S110). At this time, the profile measuring part may use a three-dimensional laser scanner.

Meanwhile, before generating the three-dimensional profile information, the interior and exterior of the sterilized bone graft may be inspected to determine whether the bone graft is normal or not, so that a normal bone graft can be used.

In addition, the three-dimensional profile information generated by the profile measuring part is provided to a controller, and the three-dimensional profile information is stored in a storage through the controller.

Further, the three-dimensional profile information stored in the storage is provided to a shaping position generator and a shaping profile generator, respectively.

The three-dimensional profile information provided to the shaping position generator is provided to determine a position of the bone graft, which can be coupled to the bone from which the affected part is removed through comparison and analysis, thereby generating shaping position information (S120).

The shaping profile generator generates shaping profile information on the basis of a position of the generated shaping position information and the three-dimensional profile information corresponding to the bone from which the affected part is removed so that the bone graft can be coupled to the bone at a selected position (S130).

The generated shaping position information and shaping profile information are stored in the storage through the controller and then provided to a shaping processor.

The shaping processor receives a driving force to shape the bone graft on the basis of the shaping position information and the shaping profile information provided to the shaping processor (S 140).

When a multi-joint robot arm of the shaping processor is moved by the provided driving force on the basis of the shaping position information, a cutting mechanism mounted on an end of the robot arm is disposed at the shaping position of the fixed bone graft.

When a high speed rotational force is provided to the positioned cutting mechanism, the cutting mechanism and the robot arm move to shape the bone graft on the basis of the shaping profile information (S 150).

At this time, the cutting mechanism continuously performs rotation movement, and is moved by the multi-joint robot arm.

As can be seen from the foregoing, a bone graft shaping system and method in accordance with the present invention have the following effects.

Using the bone graft shaping system in accordance with the present invention, a bone graft can be precisely shaped to form a predetermined gap of 0.3 to 2 mm at the grafted bone, which is preferable for bone union, enabling normal growth of the grafted bone to reduce a recovering time thereof.

In addition, the precisely shaped bone graft can be anatomically stably fixed to the bone from which the affected part is removed to minimize insertion of artificial bones and inner metal fixtures. As a result, important structures such as patient's joints, growth plates of children, etc., can be securely maintained, and operation costs and rehabilitating time can be reduced.

Further, using the bone graft shaping system in accordance with the present invention, the bone graft can be more rapidly shaped to reduce an operation time.

While the invention has been shown and described with reference to certain example embodiments thereof, it will be understood by those skilled in the art that various changes in form and details may be made therein without departing from the spirit and scope of the invention as defined by the appended claims.

## Claims

1. A bone graft shaping system comprising:
a profile measuring part configured to measure a profile of a bone graft and a profile of a bone from which an affected part is removed to generate three-dimensional profile information;
a shaping position generator configured to compare and analyze a shaping position of the bone graft corresponding to the bone from which the affected part is removed according to the generated three-dimensional profile information to generate shaping position information;
a shaping profile generator configured to generate shaping profile information to shape the bone graft according to the three-dimensional profile information corresponding to the bone from which the affected part is removed;
a shaping processor configured to shape the bone graft; and
a controller configured to control the shaping processor on the basis of the three-dimensional profile information, the shaping position information, and the shaping profile information.

2. The bone graft shaping system according to claim 1, wherein the profile measuring part is a three-dimensional scanner using a laser.

3. The bone graft shaping system according to claim 1, wherein the shaping processor comprises:
a fixing member configured to fix the bone graft; and
at least one cutting mechanism installed at a multi-joint robot arm to cut and shape the bone graft.

4. The bone graft shaping system according to claim 1, further comprising a storage configured to store the three-dimensional profile information, the shaping position information, and the shaping profile information.

5. The bone graft shaping system according to claim 4, further comprising a display configured to display at least one of the three-dimensional profile information, the shaping position information, and the shaping profile information on a screen.

6. The bone graft shaping system according to claim 1, further comprising a sterilizer configured to sterilize the bone graft and the bone from which the affected part is removed.

7. The bone graft shaping system according to claim 6, further comprising an abnormality detector configured to measure the sterilized bone graft to detect whether the bone graft is normal or not.

8. A bone graft shaping method comprising:
measuring profiles of a bone from which an affected part is removed and a sterilized bone graft, and generating three-dimensional profile information;
storing the generated three-dimensional profile information in a storage;
comparing and analyzing a shaping position of the bone graft corresponding to the bone from which the affected part is removed according to the stored three-dimensional profile information, and generating shaping position information;
generating shaping profile information to shape the bone graft according to the three-dimensional profile information corresponding to the bone from which the affected part is removed; and
shaping the bone graft on the basis of the shaping position information and the shaping profile information.

9. The bone graft shaping method according to claim 8, further comprising inspecting the interior and exterior of the sterilized bone graft to determine whether the bone graft is normal or not, before generating the three-dimensional profile information.
